# EUROPEAN PATENT APPLICATION

(11) **EP 1 810 611 A1**
(43) Date of publication of application: **25.07.2007**
(21) Application number: 06100651.6
(22) Date of filing: 20.01.2006
(51) Int. Cl.: A61B 5/022

(54) **A system for blood pressure measurement and a method for blood pressure measurement**

(71) Applicant: Microlife Intellectual Property GmbH, 9443 Widnau (CH)
(72) Inventor: Kin Yuan Lin, Taipei, 114 (TW)
(74) Representative: Müller, Christoph Emanuel

(57) **Abstract**

A system (1) for blood pressure measurement at the point of care comprises a blood pressure measuring device (10) for measuring the blood pressure of an individual (I) and seat (20) for properly positioning the individual (I). The seat (20) includes means (21) for ensuring correct position of the individual's feet and legs. The blood pressure measuring device (10) is arranged at a location (12) physically separated from a data collecting device (30). The blood pressure measuring device (10) is provided with a communication interface (11) for transmitting data to the collecting device (30).

## Description

The invention relates to a system and a method for blood pressure measurement. Hypertension is one of the major health problems in modern societies. Reliable determination of blood pressure of individuals is one of the most important conditions for appropriate treatment of hypertension. Inappropriate treatment or medication may be a result from inaccurate measurements. It has been suggested by several authors that home measurements may be most suitable for accurate blood pressure monitoring (see e.g. Laurie Barclay, "Home measurement best for accurate BP monitoring", BMJ.2002; 325:254-257 or Judy Possidente Kaufman et al., "The role of home blood pressure monitoring in hypertension control", J Clin Hypertens 3(3):171-173).

It is generally known that the so called white coat effect leads to inaccurate blood pressure measurements done by physicians. Too high blood pressure readings done by general patricians should not be used to make decisions about treatments. However, according to Kaufman, there are still some problems with self reported blood pressure measurements. One major problem is erroneous reporting. Consequently, prudent clinicians will augment home reading data with periodic clinic measurements.

According to Pickering (Pickering et al., "Recommendations for blood pressure measurement in human and experimental animals", Hypertension, January 2005, page 144,) surveys have shown that physicians and other health care providers rarely follow established guidelines for blood pressure measurements. There are several reasons why usual clinic readings may give poor results. This may not be only due to poor technique but also because they typically only consist of one or two individual measurement. However, beat to beat blood pressure variability is such that a small number of readings can only give a crude estimate of the average level. A plurality of measurements therefore will be needed in order to have reliable results.

Furthermore, measuring errors may be caused by wrong posture. In particular, back support may affect seated blood pressure determinations (see Cushman et al., "Effect of back support and stethoscope head on seated blood pressure determinations", AM J Hypertens.1990;3:240-241). Also crossing the legs may raise systolic pressure (Peters et al., Blood Press Monit.1999;4:97-101, "The effect of crossing legs on blood pressure: randomised single-blind cross over study"). Further authors (Terrent et al., "Epidemiological perspective of body position and arm level in blood pressure measurement", Blood Press.1994;3:156-163) have concluded that deviations from the recommended body position and arm level may be clinically relevant. It can be followed that reliable blood pressure measurements can only be achieved if a plurality of measurements are done with a correct posture. Furthermore, the effect of white coat hypertension needs to be considered. In the recommendations for blood pressure measurement in humans and experimental animals (Pickering, Hypertension, January 2005, page 142-161) recommendations for correct blood pressure measurements are given. In particular, the individual should be comfortably seated with legs uncrossed and back and arms supported. The patient should be seated in a chair. At an initial visit, blood pressure should be measured in both arms. Furthermore, the individual must relax before initial measurements. Furthermore, a plurality of readings is suggested in order to form more reliable averages. Reduction of white coat hypertension effect can be achieved by automatic determination of blood pressure with automatic blood pressure measurement devices.

While there are many recommendations and suggestions for blood pressure measurement, currently there is no system available for carrying out measurements in accordance with such recommendations.

It is therefore an object of the present invention to overcome the drawbacks of the prior art, in particular to provide a system and a method for blood pressure measurements which allow to make accurate and reliable blood pressure measurements. In accordance with the present invention, these and other objects are solved with a system and a method according to the independent patent claims.

According to a first aspect of the invention, there is provided a system for blood pressure measurement which is particularly suitable for measurement at a point of care. While it is generally accepted that home blood pressure monitoring may be a preferred choice, practitioners may wish to verify home measurements with there own measurement. Therefore, the present system proposes a device for point of care measurement, e.g. for measurement at a doctor's office or at a pharmacy. The system comprises a blood pressure measuring device for measuring the blood pressure of an individual. Furthermore, the system is provided with a seat for the individual adapted for properly seating the individual in view of correct blood pressure measurement. The seat includes means for ensuring a correct position of the individual's feet. This typically maybe a foot rest or also a foot pad including feet printing in order to indicate appropriate position. The system according to the present invention makes sure that measurements of blood pressure can only be done when the individual is properly positioned. By integrating or combining a blood pressure measuring device and an appropriate seat, correct position is automatically achieved.

According to a further aspect of the invention, there is provided a blood pressure measurement system which comprises a blood pressure measuring device for measuring the blood pressure of an individual. The blood pressure measuring device has a communication interface for transmission of measured blood pressure data. The system further comprises a data collection device which has a communication interface for receiving data transmitted by the blood pressure measuring device. According to the present invention, the data collecting device is arranged at the point of care but at a location physically separated from a location of the blood pressure measuring device. Physical separation is made in such a way that a direct contact between a doctor or a care person and the individual during blood pressure measurements can be prevented. By arrangement of the blood pressure measuring device at the point of care but physically separate from a care person, the white coat hypertension effect can be reduced or avoided. In particular, the blood pressure measuring device can be arranged at a separated location such as a room or corner which is particularly adapted for blood pressure measurements according to predetermined measurement criteria. In particular, there can be provided a seat as mentioned above for ensuring proper posture of the individual. Furthermore, the separated location can be designed in order to allow the individual to relax. In particular, the location may be a quiet room visually and/or acoustically isolated from the location of the data collection device or from a doctor's office.

According to a further preferred embodiment of the invention, the system comprises means for forming averages of measurement values of a plurality of subsequent measurements. This allows to further increase reliability of the results.

The system further may be provided with means for determining a difference of blood pressure values measured on the right arm and on the left arm of the individual. Inter-arm differences which may be clinically relevant (see e.g. Lane D, Beevers M, Barnes N, Bourne J, John A, Malins S, Beevers DG. Inter-arm differences in blood pressure: when are they clinically significant? J Hypertens. 2002; 20: 1089-1095) may be determined therewith.

It's preferred to measure blood pressure in both arms at the initial assessment, ideally with an automated BP device. First, this is done in order to prevent misdiagnosis of hypertension. Second, it can be ensured that the effectiveness of the therapy will be accurately monitored by measuring the BP in the same arm on all subsequent occasions.

If a reproducible difference is evident, the arm with the highest BP recording should be used in future for all BP measurements.

The system further may be designed with means for making a pause of a predetermined or predeterminable length between subsequent measurements. By making a pause, it is made sure that the individual is brought to a relaxed rest condition before a further measurement is made.

The system further may be designed for running a separate initial visit loop if a first measurement is made for a specific individual. A decision if a measurement is an initial measurement can be made by entering appropriate information into the system. The system may be provided with a specific input interface for this purpose.

According to a further preferred embodiment of the invention, the system may be provided with an input interface for entering blood pressure data during this initial visit loop. It may be preferred by some practitioners to also consider manually made blood pressure measurements, e.g. blood pressure measurements based on auscultatory measurement. These measurements will be stored in the system and will be associated to a specific individual.

The system can perform automated or auscultatory measurements. The care person can perform the auscultatoty measurement in a specific manner e.g. at the patient's initial visit.

In particular, measurements of blood pressures on the right and on the left arm and formations of differences between these measurement values will be done in the initial visit loop.

According to a further preferred embodiment of the invention, the seat of the system according to the invention includes also at least one of an arm or a back support for the individual. This further enhances proper posture of the individual.

It is further preferred for the data collection device to include a communication interface for transmitting collected blood pressure data to a central data base or a central storage means. Such an additional communication interface allows to store data of the individual in a centralised manner such that a plurality of care persons such as doctors, nurses, pharmacists or also family members can access or update the individual's data.

According to still a further preferred embodiment of the invention, the system according to the invention may include means for generating reports in accordance with predetermined reporting criteria. In particular, in view of pay for performance programs for doctors or also in view of medication therapy management programs for pharmacists, such reports might be useful. If the device is designed for automatically generating such reports, it will be much easier for the doctor or pharmacist to follow the recommendations or rules of such programs. Typically, such reports may be created as electronic files or also in paper form.

According to a further aspect of the invention, there is provided a method for blood pressure measurement of an individual at a point of care. In a first step, the individual is placed properly at a location in such a way that there is no direct contact between the individual and a doctor or a care person during blood pressure measurement. Subsequently, a blood pressure measurement for gathering blood pressure data of the individual is automatically carried out with a blood pressure measuring device in absence of a doctor or of a care person. Therewith, inaccurate measurements because of the white coat effect are avoided. The gathered blood pressure data are subsequently transmitted to a data collection device which is arranged at the point of care but at a location physically separated from the blood pressure measuring device.

According to a further preferred method of the invention, prior to the measurement of the blood pressure of the individual, one or plural instructions may be given to the individual. In particular, instructions such as to have legs and arms supported, to place feet in a predetermined manner, in particular on a predetermined foot rest or instructions to rest for a predetermined time may be given to the individual. By automatically providing such instructions, proper posture of the individual may be ensured. These instructions can be generated either automatically by the system or by a care person.]

According to still a further embodiment of the invention, prior to blood pressure measuring, in a judgement step, a judgement as to whether or not this is the individual's first or initial visit is made. Depending on the answer, different measurement routines may be appropriate. In particular, an initial visit loop can be carried out if this is the individual's initial visit. In this initial visit loop, it is e.g. possible and preferred to enter manually measured blood pressure data into the system before automatic measurements will be made. It is also possible to carry out blood pressure measurements of the individual on the individual's left and right arm during the initial visit loop in order to form respective blood pressure values. Preferably, it is also possible to form a difference between the measurements on the left and on the right arm.

If differences between the 2 arms are found, it is recommended (see page 149 of "Recommendations for Blood Pressure Measurement in Humans and Experimental Animals") that blood pressure should be checked in both arms at the first examination. This may be helpful in detecting coarctation of the aorta and upper extremity arterial obstruction. When there is a consistent interarm difference, the arm with the higher pressure should be used.

According to a further preferred embodiment of the invention, at least two subsequent measurements of blood pressure values are made. An average of measurements of subsequent readings of the blood pressure values then may be formed.

According to still a further embodiment of the invention, a pause of a predetermined or a predeterminable length is made between subsequent measurements. It is also possible to make a pause before the initial measurement in order to make sure that the individual is at rest.

When a plurality of subsequent measurements is made, it is also possible and preferred to form a difference between subsequent measurement results. If the blood pressure values of two subsequent measurements differ too much, i.e. if a difference between blood pressure values of two subsequent measurements is above a predetermined or a predeterminable value, a further measurement may be carried out in order to exclude potentially erroneous results.

The invention will now be described with respect to the following embodiments and accompanying drawings, which show:
- Figure 1: A schematic representation of a seat according to the present invention
- Figure 2: A systematic representation of a system according to the present invention
- Figures 3-5: Flow charts of operations of a method according to the present invention

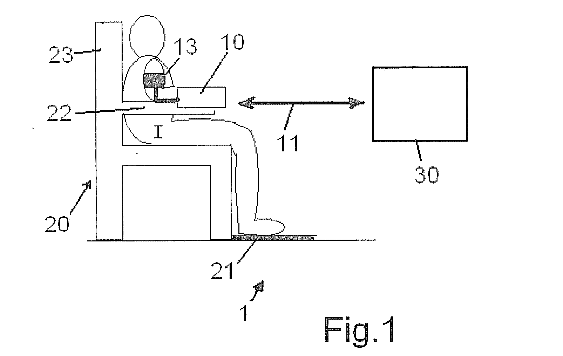

Figure 1 schematically shows a system 1 for measuring hypertension of a patient at a point of care. The system 1 basically comprises a blood pressure measuring device 10 and a seat 20 for proper positioning of the individual I. The blood pressure measuring device 10 is designed in a substantially conventional manner and includes a cuff 13 to be placed around an upper arm of the individual I. The blood pressure measuring device 10 is connected to the cuff 13 via a tube. The blood pressure measuring device 10 includes in a conventional manner a pump, pressure sensor and microprocessor means for determining automatically the blood pressure of the patient. In particular, determination may be made in the oscillometric method.

The blood pressure measuring device 10 is provided with a data communication interface 11 for transmission of data measured with the blood pressure measuring device 10 to a data collection device 30. The data collection device 30 typically is a personal computer, a personal digital assistant or another electronic equipment. It may also be a printer for printing out the measured values. Data transmission may be through a wire connection such as USB or RS 232 or preferably wireless such as by a Blue Tooth connection.

The system 1 further comprises a seat 20 for the individual I. The seat 20 is provided with foot rests 21. The foot rests 21 include markings or indications for proper positioning of the left and the right foot. These markings (not shown) make sure that the individual I is taking a measurement with uncrossed legs in accordance with measurement recommendations. The seat 20 further includes an arm support 22 and a back support 23 for ensuring proper positioning of the individual I.

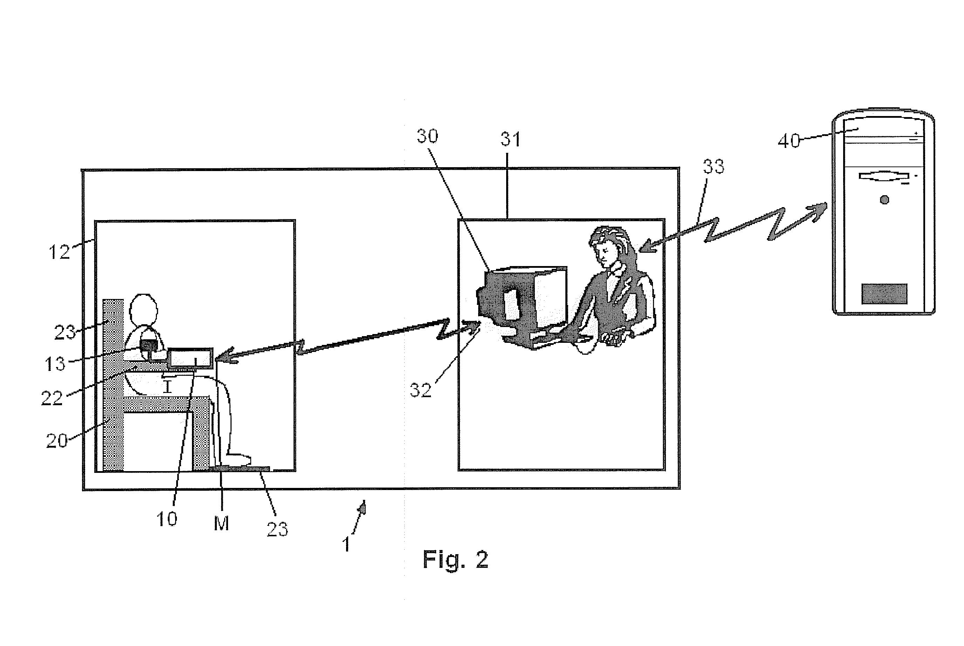

The system 1 is shown in some more detail in Figure 2, whereas like reference numerals designate like parts. The blood pressure measuring device 1 together with the seat 20 is arranged at a quiet location 12. The data communication interface 11 allows for data transmission to the data collecting device 30 in the form of a personal computer. The data collecting device is typically arranged at a doctor's office 31 physically separated from the quiet location 12. With the system according to Figure 2, blood pressure measurements may be taken with a patient properly seated on the seat 20 at the quiet location 12. Inaccurate measurements due to improper positioning or in view of white coat effect are avoided. A doctor seated in the doctor's office 31 may review a patient's file and measurement results. The measurement results may be stored in a data base. A further communication interface 33 such as an internet connection between the data collecting device 30 and an external data base server 40 may allow for centrally storing patient information including blood pressure measurement data.

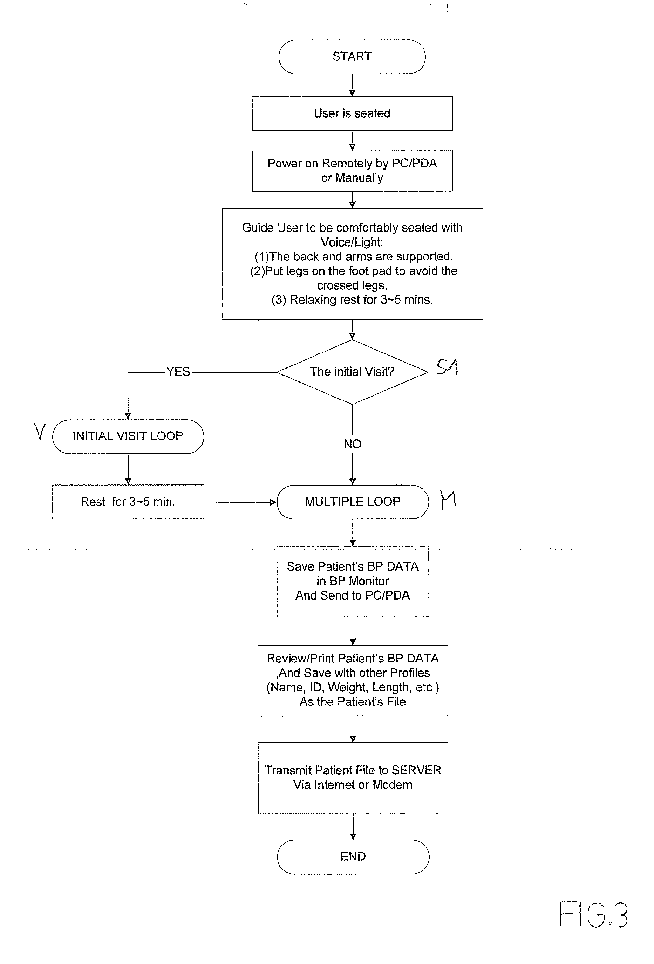

A flow chart for carrying out the method according to the present invention is shown in Figure 3. In a first step, the user or individual I is properly seated on the seat 20. The power of the system 1 is then either manually or preferably remotely turned on.

In a further step, the user is guided to be comfortably seated within the seat 20. This may be done acoustically or visually either by generating voice messages or by displaying messages on a screen. In particular, the user is instructed to have his back and arms properly supported by the back support 23 and the arm support 23. The user is further instructed to have his legs properly positioned on the foot rest 21 in order to avoid crossed legs. Furthermore, the user is instructed to relax for approximately 3 to 5 minutes. All these steps are preferably generated from a remote location or automatically in absence of a doctor or a care person.

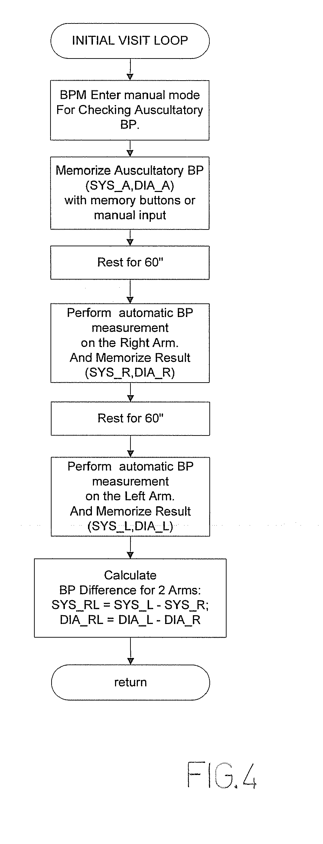

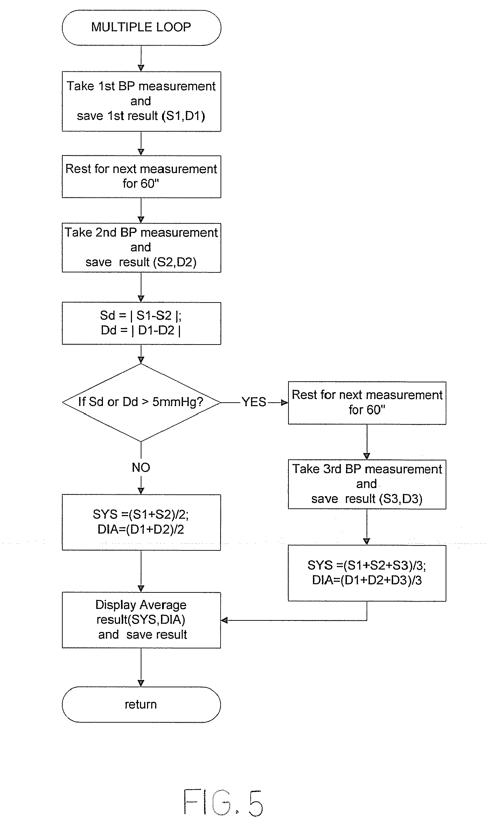

In a subsequent judgement step S1 it is judged whether this is the individual first visit. If this is the initial visit of the individual I, an initial visit loop V (see Figure 4) is carried out. After the initial visit loop V, another rest for a predetermined period of time, approximately 3 to 5 minutes will be made. After the rest, blood pressure measurements will be made in a multiple loop (see Figure 5). After completion of the multiple loop M, patient blood pressure data are stored in the blood pressure measuring device 10 and sent to the data collecting device 30 through the communication interfaces 11 and 32, respectively. In a further step, the patient's blood pressure data will be reviewed by a doctor, care person or pharmacist on the data collecting device 3 and will be saved with other relevant information such as name, ID, weight, length or the like in a patient's file. In a final, optional step, this patient file may be transferred to the central server 40 through the communication interface 33.

The initial visit loop V is shown in Figure 4. In a first step, manually measured blood pressure values are entered into the system. These are typically blood pressure values achieved in traditional auscultatory methods. The manually gathered blood pressure values SYS_A, DIA_A are stored in the system by actuating memory buttons or appropriate input buttons (not shown). After entry of these data, the individual is asked to rest for about 60 seconds. In a subsequent step, automatic blood pressure measurements will be carried out with the blood pressure measuring device 10 on the right arm. Blood pressure values SYS_R and DIA_R achieved thereby are stored in the system. Storage in particular is made within the blood pressure measuring device 10. It is, however, also possible to have the data stored at other locations.

After another rest of 60 seconds, an automatic blood pressure measurement is made on the left arm with the blood pressure measuring device 10. Typically, one cuff 13 is used for measurement on the left and on the right arm. It is, however, also possible to have two cuffs, one cuff provided for measurement on each of the arms. In this case, it will also be possible to have parallel measurements of the blood pressure on the left and on the right arm. The blood pressure values SYS_L, DIA_L measured on the left arm are subsequently stored in the system.

In a final step, blood pressure differences SYS_RL and DIA_RL are calculated between the two arms. These differences are stored in the system. These differences may be helpful in detecting coarctation of the aorta and upper extremity arterial obstruction. If there is a consistent inter-arm difference, the arm with the higher pressure should be used.

If this is not the individual's initial visit, a multiple loop is immediately carried out. In the multiple loop (see Figure 5) a first blood pressure measurement is made with the measurement device 10. The measurement results S1, D1 are stored. After a rest for 60 seconds, a second measurement is made and the results S2, D2 are stored in the system. Subsequently, an absolute value of a difference Sd, Dd is formed between the measurement values of the first and the second measurement. If Sd or Dd is below 5mmHg, an average SYS, DIA is formed of the measurement results of the first and the second measurement. These average results SYS, DIA are displayed and saved in the system. If the difference Sd, Dd is above 5mmHg, a third blood pressure measurement will be made after another rest of 60 seconds. The measurement results S3, D3 of the third measurement will be stored and average values SYS, DIA of the three measurements will be formed and displayed.

## Claims

1. A system (1) for blood pressure measurement, in particular for measurement at a point of care (POC), the system comprising
- a blood pressure measuring device (10) for measuring the blood pressure of an individual (I)
- a seat (20) for said individual (I) adapted for properly seating said individual (I) for correct blood pressure measurements,
wherein said seat includes means (21) for ensuring correct position of the individual's feet.

2. A system (1) for blood pressure measurement, in particular according to claim 1, the system comprising
- a blood pressure measuring device (10) for measuring the blood pressure of a patient, said blood pressure measuring device (10) having a communication interface (11) for transmission of measured blood pressure data,
- a data collection device (30) having a communication interface (32) for receiving data transmitted by said blood pressure measuring device,
wherein said data collection device (30) is arranged at a location (31) physically separated from a location (12) of said blood pressure measuring device (10) in such a way that a direct contact between a doctor or a care person and the individual (I) during blood pressure measurement can be prevented.

3. A system according to claim 2, wherein said blood pressure measuring device (10) is arranged at a separated location (12) at the point of care, said location (12) being adapted for blood pressure measurements according to predetermined measurement criteria.

4. A system according to one of the claims 1 to 3, wherein said system (1) comprises means for forming averages (DIS, SYS) of measurement values (S1, S2, S3; D1, D2, D3) of subsequent measurements.

5. A system according to one of the claims 1 to 4, wherein the system (1) comprises means for determining a difference (SYS_RL, DIA_RL) of blood pressure values (SYS_R, DIA_R) measured on the right arm and blood pressure values (SYS_L, DIA_L) measured on the left arm of the individual (I).

6. A system according to one of the claims 4 or 5, wherein the system (1) comprises means for making a pause of a predetermined or predeterminable length between subsequent measurements.

7. A system according to one of the claims 1 to 6, wherein the system (1) is designed for running a separate initial visit loop (V) if a first measurement is made for a specific individual (I).

8. A system according to claim 7, wherein said system (1) comprises an input interface for manually entering blood pressure data during said initial visit loop (V).

9. A system according to one of the claims 1 to 8, wherein said seat (20) includes at least one of an arm support (22) and a back support (23) for said individual (I).

10. A system according to one of the claims 2 to 9, wherein said data collection device (30) further includes a communication interface (33) for transmitting blood pressure data to a central data base.

11. A system according to one of the claims 2 to 10, wherein said system (1) includes means for generating reports in accordance with predetermined reporting criteria, particular in accordance with pay for performance criteria.

12. A method for blood pressure measurement of an individual (I) at a point of care, comprising the steps of
- placing said individual (I) at a location (12) in such a way that there is no direct contact between the individual (I) and a doctor or care person during blood pressure measurement
- automatically carrying out a blood pressure measurement of said individual (I) with a blood pressure measuring device (10) in absence of a doctor or a care person for gathering blood pressure data
- transmitting said blood pressure data to a data collection device (30) arranged at a location (31) physically separated from said blood pressure measuring device (10)

13. A method according to claim 12, wherein prior to the measurement of blood pressure data, at least one instruction selected from the group of instructions to have feet properly positioned, to have arms supported, to place feet on a predetermined foot support (21) and to take a rest for a predetermined period of time are given to the individual (I).

14. A method according to one of the claims 12 or 13, wherein prior to blood pressure measurement, in a judgment step (S1), a judgement is made as to whether or not this is the individual's (I) initial.

15. A method according to claim 14, wherein an initial visit loop (V) is carried out if this is the individual's (I) initial visit.

16. A method according to claim 15, wherein in the initial visit loop (V) manually measured blood pressure data are entered into the system (1) prior to automatic measurement.

17. A method according to claims 15 or 16, wherein in the initial visit loop (V), measurement of the blood pressure of the individual (I) are carried out on the individual's (I) left and right arm in order to determine blood pressure values (DIA_R, SYS_R) of the right arm and blood pressure values (DIA_L, SYS_L) of the left arm and wherein preferably differences (DIA_RL, SYS_RL) of the blood pressure values (DIA_R, SYS_R) of the right arm and of the blood pressure values (DIA_L, SYS_L) of the left arm are formed.

18. A method according to one of the claims 13 to 17, wherein at least two subsequent measurements are taken and wherein an average (SYS, DIA) of blood pressure values (S1, S2, S3, D1, D2, D3) of subsequent measurements is determined.

19. A method according to claim 18, wherein a pause of a predetermined or predeterminable length is made between subsequent measurements.

20. A method according to one of the claims 18 or 19, wherein a difference (Sd, Dd) between blood pressure values of two subsequent measurements is formed and wherein a further measurement is carried out if such difference (Sd, Dd) is above a predetermined or predeterminable value.
